# EUROPÄISCHE PATENTSCHRIFT

(11) **EP 0 364 783 B1**
(45) Veröffentlichungstag und Bekanntmachung des Hinweises auf die Patenterteilung: **03.11.1993**
(21) Anmeldenummer: 89118012.7
(22) Anmeldetag: 28.09.1989
(51) Int. Cl.: B65D 51/00, A61J 1/00

(54) **Verschluss für eine Medikamentenflasche und Verfahren zur Herstellung dieses Verschlusses**
Closure for a medical bottle, and method of producing the same
Fermeture pour une bouteille à usage médical et son procédé de préparation

(30) Priorität: 20.10.1988 DE 3835720
(43) Veröffentlichungstag der Anmeldung: 25.04.1990
(73) Patentinhaber: Pharma-Gummi Wimmer West GmbH, 52249 Eschweiler (DE)
(72) Erfinder: Wimmer, Hans, D-5100 Aachen (DE)
(74) Vertreter: Patent- und Rechtsanwaltssozietät, Schmitt, Maucher & Börjes

(56) Entgegenhaltungen:
- BE-A- 895 575
- CH-A- 485 463
- DE-A- 2 327 553
- DE-A- 2 844 206
- GB-A- 695 839
- GB-A- 1 266 302
- US-A- 2 135 386
- US-A- 3 326 401

## Beschreibung

Die Erfindung betrifft einen Verschluß für eine Medikamentenflasche nach dem Oberbegriff von Anspruch 1. Die Erfindung befaßt sich auch mit einem Verfahren zum Herstellen eines Verschlusses für Medikamentenflaschen gemäß dem Oberbegriff von Anspruch 13.

Man kennt bereits verschiedene Verschlüsse dieser Art, insbesondere für Infusionsflaschen. Diese bekannten Verschlüsse weisen jedoch noch erhebliche Nachteile auf. Solche Verschlüsse sind auf den zu verschließenden Flaschenhals aufsetzbar und haben z. B. eine durchstechbare, mit einer Einstichmarkierung versehene Dichtungsscheibe (vgl. DE 24 21 359 C 2; DE 33 10 265 A 1 u. DE 35 44 109 C 2). Bei solchen Verschlüssen wird das Dichtelement, das vorgeformt in eine Kappe des Verschlusses eingelegt wird, durch Auf- oder Abreißen eines Kappendeckels od. dgl. freigelegt. In DE-PS 23 27 553 ist ein in der Verschlußkappe geformtes Spritz- oder Gießteil beschrieben, wobei das auf- bzw. abreißbare Deckelteil auf seiner dem Dichtelement zugewandten Seite mindestens ein Formteil hat, dessen Gestalt der gewünschten Ausformung als Formstück entspricht.
Nachteile der bekannten, vorbeschriebenen Verschlüsse betreffen insbesondere ihre biologische Sicherheit und eine nicht genügend bequeme und sichere Handhabung. In aller Regel wird nämlich der Inhalt einer solchen Flasche mit Hilfe eines Hohldorns oder einer Kanüle entnommen, wobei der Hohldorn od. dgl. durch das Dichtelement durchgestochen wird. Dazu muß bei den vorerwähnten Verschlüssen zunächst der Deckel der Verschlußkappe auf- bzw. abgerissen werden, damit ein durchstechbarer Teil des Dichtelementes freigelegt wird. Dieses Auf- bzw. Abreißen des Deckels führt bei fehlerhafter Handhabung häufig zum Abreißen des angeformten Reißringes, ohne daß die Durchstichstelle freigelegt wird. Dann kann der Flascheninhalt nicht mehr entnommen werden.
Außerdem bestehen die vorbekannten Verschlüsse für Medikamentenflaschen häufig aus zwei Teilen, nämlich der eigentlichen äußeren, topfförmigen Verschlußkappe und einer darin eingelegten Dichtscheibe aus elastischem Werkstoff. Daraus ergibt sich der Nachteil, daß zwischen diesen beiden Teilen ein wenn auch kleinvolumiger Trennbereich und somit wenigstens ein Hohlraum verbleibt, in der sich Unsauberkeiten, Fremdpartikel od. dgl. festsetzen können, da das Einsetzen der Dichtscheibe in die Verschlußkappe nicht unter sterilen Bedingungen erfolgt. Insbesondere bei Medikamentenflaschen ist dies äußerst unerwünscht und man muß die Flasche mit dem aufgesetzten Verschluß in einem teueren und aufwendigen Prozeß sterilisieren oder thermisch nachbehandeln. Dies hat in der Regel wiederum den Nachteil, daß die Kappe über die Umwandlungstemperatur des Kappenmaterials erhitzt werden muß, wobei es unter der Druckspannung des eingelegten gummielastischen Dichtelementes zu Verformungen dieser Kappe kommen kann, die dann beim späteren Gebrauch, insbesondere bei Entnahme des Flascheninhaltes unter Druck (Druckinfusion) zu Undichtigkeiten führen kann.

Ein weiterer Nachteil der vorerwähnten Verschlüsse besteht darin, daß nach dem Auf- oder Abreißen des Kappen-Deckels die Stirnfläche der Kappe und die Oberfläche der Dichtscheibe nicht in der gleichen Ebene liegen. Vor dem Durchstechen eines Hohldornes ist es jedoch üblich, die Durchstichstelle mit einem geeigneten Desinfektionsmittel zu desinfizieren. Durch die unterschiedliche Lage der Flächenebenen von Kappen- und Dichtelement-Stirnseite ist jedoch eine einfach durchzuführende, saubere Desinfektion des Durchstichbereiches nicht gewährleistet.
In DE-PS 23 27 553 ist ein Verschluß mit einer elastischen Dichtschicht beschrieben, der eine mittels einer Grifflasche abziehbare, im ungebrauchten Zustand geschlossene Oberseite hat. Das dort beschriebene Verfahren vermag zwar unter Umständen die Probleme der Dichtigkeit zu verbessern, es bleiben jedoch die anderen vorbeschriebenen Probleme bzw. Nachteile.

Man kennt auch bereits eine Schutzkappe für einen eine zu sterilisierende Lösung enthaltenden Kunststoffbehälter (DE 25 09 504 B 2). Dort ist auch bereits ein Verschluß für eine Medikamentenflasche der eingangs erwähnten Art gezeigt, bei der eine aus thermoelastischem Kunststoff bestehende Dichtungsscheibe bei einer Sterilisationstemperatur erweicht und der verschlossenen Stirnseite des aus thermoplastischem Kunststoff bestehenden Kappengehäuses nachgeformt wird. Mit einer solchen Schutzkappe wird die Schaffung eines Medikamentenverschlusses angestrebt, dessen Dichtungsscheibe an der Stirnseite der Kappe und an der Stirnseite des Behälters ohne Bildung von Hohlräumen Konturgetreu und fest abdichtend anliegt, so daß Bakterienherde und Flüssigkeitsansammlungen unter der Dichtungsscheibe wenigstens weitgehend vermieden werden. Mit einem solchen Verschluß erreicht man zwar ein Anschmiegen und mehr oder weniger dichtes Anformen der dort als Dichtelement dienenden Dichtungsscheibe an die ihr benachbarten Wände des Kappengehäuses. Eine große biologische Sicherheit ist jedoch, insbesondere auch beim Herstellen von großen Stückzahlen dieser Schutzkappe, nicht mit der gewünschten Zuverlässigkeit gewährleistet und es fehlt an einer genügend bequemen und sicheren Handhabung beim Gebrauchsfertig-Machen dieser Schutzkappe, besondere bezüglich ihres Einstichbereiches für einen Hohldorn oder eine Kanüle. Das angestrebte dichte Anformen benachbarter Verschlußteile schließt nämlich Verunreinigungen im Trennbereich zwischen der Schutzkappe 10 und der Dichtungsscheibe nicht vollständig aus. Außerdem ist man bei der Wahl des thermoplastischen Werkstoffes für die Dichtungsscheibe erheblich eingeengt, da dieser Werkstoff sich einerseits bei der Sterilisationstemperatur erweichen und an die benachbarte Kontur der Schutzkappe anpassen soll, selbst aber keine Schäden bei der Temperatursterilisation erleiden darf. Außerdem muß dieser Dichtungsscheiben-Werkstoff so geartet sein, daß beim Abkühlen von der Sterilisationstemperatur zur Gebrauchs- oder gar Unterkühlungs-Temperatur keine zu großen Schrumpfspannungen auftreten. Die Gebrauchstemperatur ist bei solchen Verschlüssen in der Regel die Zimmertemperatur. Bei bestimmten Medikamentenflaschen kann auch die Forderung hinzukommen, daß Verschluß und Medikamentenflasche über längere Zeit unterkühlt aufbewahrt werden müssen.

Aus der dem Oberbegriff des Anspruchs 1 zugrundeliegenden DE-OS 28 44 206 kennt man auch bereits einen Verschluß der eingangs erwähnten Art, der im wesentlichen aus zwei oder drei miteinander verbundenen Einzelteilen besteht. Zwar ist auch dort bereits der Verschluß für die Medikamentenflasche mit einer Kappe versehen, die an ihrer Stirnseite wenigstens eine Durchstichöffnung aufweist, in welche jeweils ein Teil einer elastischen Dichtschicht hineinragt, wobei die Kappe aus einem im Vergleich zum Dichtelement weniger elastischen Werkstoff besteht und ihre äußere Stirnfläche zumindest die Durchstichöffnung überdeckende, entfernbare Abdeckung hat. Eine zuverlässige Abdichtung und störunempfindliche Handhabung bietet auch dieser Verschluß nach DE-OS 28 44 206 nicht. Bei einem Ausführungsbeispiel weist die dortige Kappe an der Stirnwand ein kreisförmiges Wandteil auf, welches mit Hilfe eines Aufreißringes entfernbar ist.

Dementsprechend besteht wiederrum die Gefahr, daß es bei einer ungeschickten Handhabung dazu kommt, daß zwar der Aufreißring abgerissen wird, jedoch das für die Freilegung der elastischen Dichtschicht notwendigerweise zu entfernende mittlere Kappen-Stirnwandteil trotz einer Schwächungslinie nicht zusammen mit dem Aufreißring entfernt wird. Vorallem besitzt diese vorbekannte Kappe den Nachteil, daß das Dichtteil nur in die Kappe eigesetzt ist. Eine sichere Verbindung zwischen Dichtteil und Kappe, die insbesondere auch dann zuverlässig dicht bleibt, wenn ein Hohldorn oder dergleichen durch die Dichtschicht in das Innere der Medikamentenflasche getrieben wird, ist hier ebenfalls nicht gegeben. Nicht selten kann dann die scheibenartige, elastische Dichtschicht in Richtung des Inneren der Medikamentenflasche geringfügig ausweichen. Dies geschieht erfahrungsgemäß selbst dann, wenn das Mundstück der gewöhnlich aus Kunststoff bestehenden Medikamentenflasche von der Flaschenseite her an der inneren Stirnseite des Dichtelementes anliegt. Wie die Praxis gezeigt hat, biegen sich dann oft die mundstückseitige Stirnwand der Medikamentenflasche zusammen mit der elastischen, scheibenförmigen Dichtschicht dieses vorbekannten Verschlusses zum Flascheninneren durch. Diese Verformungsbewegung begünstigt zusätzlich, daß die scheibenartige Dichtschicht bei ihren Seitenrändern gegenüber der Kappe ihre Dichtwirkung verliert. Besonders dann, wenn die Medikamentenflasche in der Gebrauchsstellung mit nach unten weisendem Verschluß aufgehängt ist, was bei einer Verwendung als Infusionsbehälter oder dergleichen regelmäßig der Fall sein wird, kann dann über die vom eingesteckten Hohldorn in die Stirnwand der Medikamentenflasche eingebrachte Lochung, die aufgrund des Medikamentenflaschen-Werkstoffes nicht dicht ist, zumindest auf dem Kriechwege etwas vom Inhalt der Medikamentenflasche entlang einer Undichtigkeitsstelle am Rande der scheibenförmigen, elastischen Dichtschicht und deren Stirnseiten zur Einstecköffnung des Dornes und von dort ins Freie gelangen. Dies ist höchst unerwünscht, da dadurch ein Teil des Inhaltes der Medikamentenflasche verloren geht.

Noch ungünstiger ist dabei aber, daß auf dem gleichen Wege über die ins Freie herauskriechende Flüssigkeit von außen her Bakterien oder dergleichen in den Flascheninhalt hineingelangen können. Nach einer abgewandelten Ausführungsform des vorbeschriebenen Verschlusses nach DE-OS 28 44 206 ist bereits vorgesehen, daß die Kappe an ihrer Stirnseite wenigstens ein Durchstichöffnung aufweist und daß diese Löcher durch den Werkstoff der Dichtungsscheiben ausgefüllt sind. So soll eine dichtere Verbindung zwischen der Dichtschicht einerseits und der Kappe andererseits erreicht werden.
Bei anderen Ausführungsformen dieser Schutzkappe sind die Durchstichöffnungen der Kappe als - vor ihrer Bereitstellung geschlossene - Höhlungen ausgebildet, jedoch wirken derartige Hohlräume im Verschluß der angestrebten biologischen Sicherheit dieses Verschlusses entgegen.

Es besteht daher die Aufgabe, einen Verschluß der eingangs beschreibenden Art hinsichtlich seiner biologischen Sicherheit bei einfacher Herstellbarkeit und guter Anwendungssicherheit zu verbessern.

Die erfindungsgemäße Lösung dieser Aufgabe besteht bei einem Verschluß der eingangs erwähnten Art darin, daß die Kappe und die Dichtschicht kappeninnenseitig untrennbar miteinander verschweißt sind. Bei einem solchen Verschluß erreicht man eine praktisch vollkommen dichte Verbindung zwischen der Dichtschicht einerseits und der dazu benachbarten Kappeninnenseite andererseits. Es wird auch vermieden, daß der Flascheninhalt zumindest teilweise z.B. auf dem Kriechwege nach außen gelangen kann. Auf diese Weise wird ein hohes Maß an biologischer Sicherheit erreicht.

Um in die Medikamentenflasche auch mehrer Hohldorne oder dergleichen einführen zu können, ist es vorteilhaft, wenn an der Stirnseite der Kappe mehrere Durchstichöffnungen vorgesehen sind.

Eine besonders vorteilhafte Weiterbildung gemäß der Erfindung sieht vor, daß mindestens eine Durchstichöffnung an der Stirnseite der Kappe mit der elastischen Dichtschicht derart verschlossen ist, daß der in diese Öffnung hineinragende Dichtschichtabschnitt mit der äußeren Stirnfläche der Kappenstirnseite einer praktisch ebene Fläche bildet. Man kann dann leicht und sicher die entsprechenden Durchstichstelle vor dem Einstoßen eines Hohldorns oder dergleichen mit einem Desinfektionsmittel überstreichen; der Desinfektion schlecht zugängliche Nischen werden vermieden und die biologische Sicherheit sowie die bequeme und sichere Handhabung noch verbessert.

Vorteilhaft ist es, wenn der oder die in die Durchstichöffnung(en) der Kappe hineinragende(n) Abschnitt(e) der elastischen Dichtschicht am Öffnungs-Wandbereich fest anliegt (anliegen) und dort untrennbar mit der Kappe verschweißt ist (sind). Dadurch erhält man auch noch im Wandbereich dieser Durchstichöffnungen eine feste, untrennbare Verbindung zwischen der dortigen Kappenwand-Oberfläche und der ihr benachbarten elastischen Dichtschicht. Dementsprechend werden auch dort kleinste Zwischenräume zwischen diesen beiden Verschlußteilen ausgeschlossen. Man erhält einen Verschluß mit sehr hoher biologischer Sicherheit, der praktisch einstückig ausgebildet ist, jedoch aus unterschiedlichen Werkstoffen besteht. Durch das durchgehende Verschweißen von Kappe und elastischer Dichtschicht auf allen gemeinsamen Berührungsflächen kann ausgeschlossen werden, daß während der Anwendung der Flascheninhalt auf dem Kriechwege zwischen elastischer Dichtschicht und der Kappe nach außen dringt.

Die Erfindung betrifft auch ein Verfahren zur Herstellung eines Verschlusses für Medikamentenflaschen, insbesondere eines Verschusses der vorbeschriebenen Art. Es ist bereits aus DE-OS 23 27 553 ein Verfahren zur Herstellung eines Verschlusses für Medikamentenflaschen bekannt, bei dem in einem Herstellungsschritt die Kappe des Verschlusses, vorzugsweise zusammen mit wenigstens einer Anzeigestelle für einen späteren Durchstichbereich, hergestellt und anschließend in einem weiteren Herstellungsschritt in dem selben Werkzeug oder einem Teil davon eine elastische Dichtschicht in die Kappe eingespritzt wird. Bei diesem Verfahren ist zwar vorgesehen, daß beim Einspritzen der Dichtungsscheibe die Temperatur des Spritzvorganges so hoch gewählt wird, daß an der Dichtungsscheibe sterile Oberflächen entstehen, gleichzeitig wird bei diesem Verfahren aber darauf geachtet, daß der Deckelteil von der Oberfläche der Dichtungsscheibe ohne Festhaften gelöst werden kann. Beim vorbekannten Verfahren erreicht man also zwar beim Einspritzen der späteren Dichtungsscheibe eine Sterilisation u. a. der benachbarten Oberflächen von Dichtungsscheibe und Verschlußkappe, es verbleibt aber eine -wenn auch noch so dünne- Trennzone, in denen Verunreinigungen, Unsauberkeiten, Fremdpartikel od. dgl. sich festsetzen können. Auch können solche kleinvolumige Trennbereiche die Ursache von Mikroorganismen-Wachstum werden. Außerdem hat der nach diesem Verfahren hergestellte Verschuß nach dem Entfernen eines Deckelteils eine dellenartige Anzeigestelle für den späteren Durchstichbereich bei der elastischen Dichtschicht. Um den Durchstichbereich freizulegen, muß aber ein Teil der Kappen-Stirnseite entfernt werden, was wegen der Stabilität des Kappenwerkstoffes nicht sehr bequem erfolgen kann. Außerdem ist dann die freigelegte Fläche des Durchstichbereiches kugelkalottenförmig zum Flascheninneren hin eingezogen, so daß ein Sterilisieren z. B. mit einem Wattebausch und Sterilisationsflüssigkeit nicht bequem zu handhaben ist, da die Oberfläche des Einstichbereiches merkbar von der von außen zugänglichen Oberfläche der Kappenstirnseite abweicht. Dabei treten die bereits erwähnten Nachteile auf.

Es besteht daher die Aufgabe, ein mit möglichst wenig Aufwand verbundenes Verfahren zum Herstellen eines Verschlusses für Medikamentenflaschen zu schaffen, bei dem sich unter Vermeidung der Nachteile vorbekannter Verfahren dieser Art insbesondere die biologische Sicherheit und die Handhabung dieses Verschlusses verbessern läßt.

Die erfindungsgemäße Lösung dieser Aufgabe besteht bei einem Verfahren zum Herstellen eines Verschlusses für Medikamentenflaschen gemäß dem Oberbegriff von Anspruch 13 darin, daß die Dichtschicht und die Kappe kappeninnenseitig untrennbar miteinander verschweißt werden. Ein solches Herstellungsverfahren ist vergleichsweise einfach und mit geringem Werkzeugaufwand durchzuführen. Vorallem wird dabei auch ein Trennbereich von noch so kleinem Volumen vermieden und bei dem Eindringen der elastischen Dichtschicht und ihrem Verschweißen findet nicht nur ein Sterilisieren des Verschlusses in diesem Bereich statt, sondern man erhält einen gewissermaßen einstückigen Verschluß, der jedoch aus zwei unterschiedlichen Werkstoffen besteht.

Zusätzliche Weiterbildung der Erfindung sind in weiteren Unteransprüchen aufgeführt.

Weitere Merkmale der Erfindung ergeben sich aus der folgenden Beschreibung von bevorzugten Ausführungsbeispielen der Erfindung in Verbindung mit den Ansprüchen und der Zeichnung. Die einzelnen Merkmale können je für sich oder zu mehreren bei einer Ausführungsform der Erfindung verwirklicht sein. Es zeigen in vergrößertem Maßstab :
- Fig. 1: eine teilweise im Längsschnitt dargestellte Seitenansicht des oberen Teils einer Medikamentenflasche mit Verschluß,
- Fig. 2: eine im teilweisen Längsschnitt dargestellte Seitenansicht einer etwas abgewandelten, bevorzugten Ausführungsform des Verschlusses ähnlich dem nach Fig. 1, und
- Fig. 3: eine Teilaufsicht auf die in Fig. 2 dargestellte Kappe.

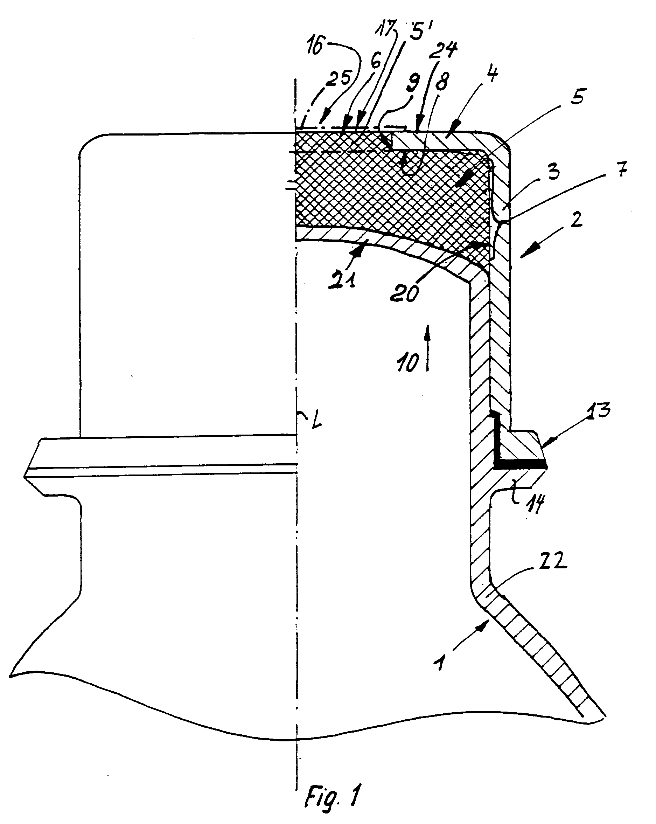

Eine aus Kunststoff bestehende Medikamentenflasche 1 hat an ihrer Flaschenmündung 10 einen diese Mündung abschließenden Flaschendeckel 21. Dieser steht einstückig mit der übrigen Flaschenwand 22 in Verbindung. Im Bereich der Flaschenmündung 10 befindet sich der im ganzen mit 2 bezeichnete Verschluß. Dabei besteht die Kappe aus einem weniger elastischen Werkstoff und das darin befindliche Dichtelement aus einer elastischen Dichtschicht, die an einem wenigstens etwa zylindrischen Teilbereich der Kappen-Innenfläche 20 sowie an der Innenfläche 8 der Stirnseite 4 der Kappe 3 fest anliegt. An der Stirnseite 4 der Kappe 3 ist ein Durchstichbereich 16 zum Einstoßen eines Hohldorns od. dgl. vorgesehen. Dieser Durchstichbereich 16 liegt bei der Ausführung nach Fig. 1 zentral und im Bereich der Längsachse L des Verschlusses.

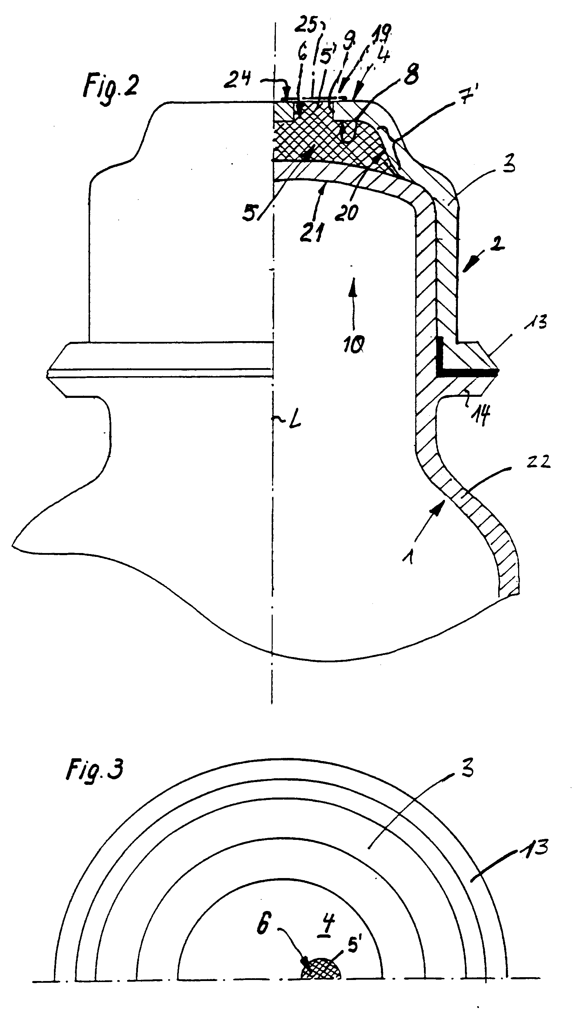

Gemäß der Erfindung ist vorgesehen, daß die Kappe 3 und die Dichtschicht 5 kappeninnenseitig untrennbar miteinander verschweißt sind. In der Stirnseite 4 der Kappe 3 ist wenigstens eine Durchstichöffnung 6 vorgesehen , in welche die Dichtschicht 5 hineinragt. Aus Fig. 2 und 3 ist erkennbar, daß Durchstichöffnungen 6 auch exzentrisch zur Kappen-Längsachse L angeordnet sein können und bei einer solchen Ausführung ist es besonders einfach, an der Stirnseite 4 der Kappe 3 mehrere Durchstichöffnungen 6 vorzusehen. Die Durchstichöffnungen 6 an der Stirnseite 4 der Kappe 3 sind mit der elastischen Dichtschicht 5 gefüllt und verschlossen. Dabei ist es besonders vorteilhaft, wenn die in diese öffnungen 6 hineinragenden Dichtschicht-Abschnitte 5' mit der äußeren Stirnfläche 24 der Kappenstirnseite 4 eine ebene Fläche bilden. Die elastische Dichtschicht 5 liegt an einem axialen, wenigstens etwa zylindrischen Teilbereich 7 der Kappeninnenseite 20 sowie der Innenfläche 8 der Stirnseite 4 der Kappe nicht nur dicht an, sondern ist dort auch untrennbar mit der Kappe 3 verbunden. Bei einer etwas abgewandelten Ausführungsform gemäß Fig. 2 erkennt man, daß der axiale, wenigstens etwa zylindrische Teilbereich 7', wo die elastische Dichtschicht 5 seitlich fest und untrennbar mit der Kappen-Innenfläche 8 verbunden ist, auch eine konische Ausbildung haben kann. Die Durchstichöffnungen 6 an der Stirnseite 4 der Kappe 3 sind -wie erwähntmit dort hineinragenden Abschnitten 5' der Dichtschicht 5 verschlossen, und zwar derart, daß die in diese Öffnungen 6 hineinragenden Dichtschicht-Abschnitte 5' mit der äußeren Stirnfläche 24 der Kappen-Stirnseite 4 eine praktisch ebene, durchgehende Fläche bilden. Man erkennt gut, daß die nach außen gewandten Stirnseiten 17 der Abschnitte 5' der Dichtschicht und die übrige, von der Kappe 3 gebildete äußere Stirnfläche 24 in einer Ebene liegen. Beim überstreichen der äußeren Stirnfläche 24 mit einem Desinfektionsmittel verbleiben keine Nischen od. dgl. schlecht erreichbare Bereiche beim Verschluß 2.

Die Abschnitte 5' der elastischen Dichtschicht 5, die in die Durchstichöffnungen 6 hineinragen, liegen dort nicht nur fest am Wandbereich 9 dieser Durchstichöffnungen 6 an, sondern sind an diesem öffnungs-Wandbereich 9 untrennbar mit der Kappe 3 verbunden, vorzugsweise verschweißt.

Aus der Zeichnung ist gut erkennbar, daß die Stirnseite 4 der Kappe 3 etwa im Bereich der Flaschenmündung 10 napfartig sowie von der Flaschenmündung 10 wegweisend ausgebildet und daß bei der Kappe 3 an ihrer der Flasche 1 zugewandten Seite ein Befestigungsflansch 13 vorgesehen ist. Dieser kann mit einem passend angeordneten Gegenflansch 14 der Medikamentenflasche 1 dichtend verbunden werden, vorzugsweise durch Verschweißen. Die Mündung 10 der Medikamentenflasche 1 ist im unbenutzten Zustand mittels eines Flaschendeckels 21 einstückig mit der Flaschenmündung bzw. der Flaschenwand 22 verbunden. Dementsprechend ist das Flascheninnere im unbenutzten Zustand vollkommen dicht abgeschlossen. Die dem Flaschendeckel 21 zugewandte Seite der elastischen Dichtschicht 5 ist in ihrer der Flasche 1 zugewandten Umrißform so an diesen Flaschendeckel 21 angepaßt, daß sie im unbenutzten Zustand dichtend daran anliegt. Daraus ergibt sich auch ein guter Schutz gegen Eindringen von Verunreinigungen nach dem Fertigstellen einer gefüllten und abgestellten Flasche.

Gemäß einer vorteilhaften Weiterbildung der Erfindung ist eine oder sind vorzugsweise alle Durchstichoffnungen 6 und die sie ausfüllenden Abschnitte 5' der elastischen Dichtschicht 5 mit einer abziehbaren, im ungebrauchten Zustand dem Umgebungsbereich 19 der Durchstichöffnung 6 dicht verschließenden Abdeckung 25 versehen. Diese ist in Fig. 1 u. 2 mit stark vergrößerter Dicke strichliniert eingezeichnet. Wenn mehrere Durchstichöffnungen 6 in der Kappe 3 vorhanden sind, können sowohl mehrere einzelne Abdeckungen 25 jede der Durchstichöffnungen 6 separat verschließen; oder es kann eine gemeinsame, vorzugsweise alle Durchstichöffnungen 6 verschließende Abdeckung 25 vorgesehen sein. Eine oder mehrere Abdeckungen 25 können entweder rund um eine oder um mehrere Durchstichöffnungen 6 auf die äußere Stirnseite 4 der Kappe 3 aufgeklebt sein. In entsprechenden Zuordnungen können auch eine oder mehrere Abdeckungen 25 rund um eine oder mehrere Durchstichöffnungen 6 auf die Stirnseite 4 der Kappe 3 aufgeschweißt sein. Die Abdeckung(en) 25 können aus PE (Polyethylen) oder PP (Polypropylen) bestehen. Die Abdeckung 25 kann auch aus einer Aluminiumfolie od. dgl. Folienwerkstoff bestehen. Zweckmäßigerweise ist an jeder Abdeckung zur leichten Handhabung eine Abreißlasche vorgesehen. Die vorerwähnten Abreißlaschen schützen den Bereich der Durchstichöffnung vor Verschmutzung, Kontamination usw., insbesondere auch während der Lagerzeit. Dabei sind diese von der Kappe 3 separaten, gegenüber der Wanddicke der Kappe 3 auch wesentlich dünneren, folienartigen Abdeckungen 25 sehr bequem, z. B. mit Hilfe der üblichen Abreißlasche bei Ingebrauchnahme der Medikamentenflasche zu entfernen. Wenn in zeitlichem Abstand in unterschiedliche Durchstichöffnungen ein Hohldorn od. dgl. eingebracht werden soll, kann man z. B. auch zuerst eine erste, separate Abdeckung 25 entfernen und im Bedarfsfalle, wenn eine weitere Durchstichöffnung 6 benutzt werden soll, die dazugehörige weitere Abdeckung 25.

Es ist vorteilhaft, wenn der Werkstoff der Dichtschicht 5 farblich gegenüber dem Kappenwerkstoff kontrastiert. Auf diese Weise lassen sich ohne zusätzlichen Aufwand die Durchstichöffnungen an der äußeren Stirnfläche 24 der Kappe kennzeichnen.

Ein besonders vorteilhaftes Herstellungsverfahren für den vorbeschriebenen Verschluß besteht darin, daß beim ersten Herstellungsschritt die Kappe 3 mit wenigstens einer Durchstichöffnung 6 hergestellt und in einem zweiten Herstellungsschritt die elastische Dichtschicht 5 in die Kappe 3 sowie deren Durchstichöffnungen 6 eingebracht sowie dort fest und untrennbar mit dem Kappenmaterial verschweißt wird.

Gemäß einer vorteilhaften Weiterbildung des Herstellungsverfahrens können eine oder mehrere Abdeckungen 25 für die Durchstichöffnung(en) 6 vor dem Einspritzen des weniger elastischen Kappen-Werkstoffes in das Werkzeug eingelegt werden. Während des Einspritzens und der Formgebung des heißen Kunststoffmaterials verbinden sich dann die Abdeckung(en) 25 mit der so entstehenden Kappe 3 fest, jedoch lösbar im Bereich der stirnseitigen Durchstichöffnungen 6 der Kappe 3. Bei einer derartigen Herstellungsweise bilden diese Abdeckungen 25 auch an der äußeren Stirnfläche 24 den Abschluß der Durchstichöffnungen 6, wenn die elastische Dichtschicht in die Kappe sowie deren Durchstichöffnungen 6 eingebracht wird.

Eine gute biologische Sicherheit erreicht man, wenn durch das vorbeschriebene Verfahren bei dem Verschluß 2 die Dichtschicht 5 mit den ihr benachbarten Bereichen der Kappe 3 durchgehend und unterbrechungsfrei miteinander verbunden ist, vorzugsweise durch Verschweißen. Das Einbringen der elastischen Dichtschicht 5 bzw. das Verschweißen erfolgt in aller Regel bei Temperaturen, die auch eine Sterilisation in diesem Bereich mitbewirken. Bei einer so vollständigen und unterbrechungsfreien Verbindung zwischen elastischer Dichtschicht 5 und Kappe 3 verbleibt praktisch keine Möglichkeit für das Einlagern von Verunreinigungen od. dgl. Fremdkörper. Auch ist ein Mikroorganismen-Wachstum in diesen für eine spätere Sterilisation des gesamten Verschlusses nicht mehr zugänglichen Bereich mit einem Höchstmaß an Sicherheit ausgeschlossen.

Bezüglich des zylindrischen Teilbereiches 7 der Kappe 3, in dem die elastische Dichtscheibe 5 an der Innenfläche 20 der Kappe 3 anliegt und z. B. mit ihr verschweißt ist, kommt es auf eine gewisse axiale Erstreckung entsprechend der notwendigen Dicke dieser Dichtschicht 5 an. Ob die Kappe 3 dort etwa zylindrisch (Fig. 1) oder leicht konisch (Fig. 2) oder in anderer Weise geformt ist, ist dagegen zweitrangig.

Als Werkstoff für die Flasche 1 und die Kappe 3 des Verschlusses kommen beispielsweise Polyethylen oder Polypropylen in Frage. Kappen- und Flaschenwerkstoff sind aufeinander abgestimmt, so daß sie an den Flanschen 13, 14 miteinander verschweißt werden können. Als Werkstoff für die elastische Dichtschicht kommt ein thermoplastisches Elastomer (TPE) bevorzugt in Frage.

Die hier dargestellten Verschlüsse lassen sich folgendermaßen herstellen : In eine Herstellungsform für die Kappe 3 wird zunächst vor dem Einspritzen des weniger elastischen Kappen-Werkstoffes eine Abdeckung 25 eingelegt, oder es werden mehrere Abdeckungen 25 dort eingebracht. Dann erfolgt nach Verschluß dieses Herstellungswerkzeuges das Einspritzen des weniger elastischen Werkstoffes, aus dem die Kappe 3 gebildet ist. Das Werkzeug ist so ausgebildet, daß die Durchstichöffnung(en) 6 der Kappe 3 mit-angefertigt werden. Während des Einspritzens und der Formgebung des heißen Kunststoffmaterials für die Kappe 3 verbindet sich die Abdeckung 25 mit dieser Kappe 3 fest, jedoch lösbar. Bei der zum besseren Ablösen dienenden Abreißlasche für die Abdeckung 25 kann im Griffbereich, z. B. durch eine der Verbindung von Abdeckungs-Lasche und Kappe entgegenwirkende Werkstoffschicht, dafür gesorgt werden, daß die Grifflasche sich nicht mit dem heißen Kunststoffmaterial der Kappe 3 zu fest verbindet. Nach diesem ersten Herstellungsschritt betreffend die Kappe 3 des Verschlusses 2 kann dann, vorzugsweise im Unterteil des Spritzwerkzeuges, die elastische Dichtschicht 5 in die Kappe eingespritzt werden. Dabei findet eine Verschweißung der elastischen Dichtschicht 5 innenseitig in der Kappe 3 statt, wobei durch die Temperatur des Einspritzvorganges gleichzeitig ein Sterilisationsvorgang mit-stattfindet.

## Patentansprüche

1. Verschluß (2) für eine Medikamentenflasche (1) mit einer topfförmigen und mit der Flasche verbindbaren Kappe (3) die an ihrer Stirnseite wenigstens eine Durchstichöffnung (6) aufweist, mit einem in der Kappe (3) befindlichen, durchstechbaren Dichtelement (5), welches aus einer elastischen, in die Durchstichöffnung (6) hineinragenden Dichtschicht (5) besteht sowie mit zumindest einer entfernbaren Abdeckung (25), die an der äußeren Stirnfläche (24) des Verschlusses (2) die Durchstichöffnung (6) überdeckt, wobei die Kappe aus einem weniger elastischen Werkstoff als das Dichtelement besteht, **dadurch gekennzeichnet,** daß die Kappe (3) und die Dichtschicht (5) kappeninnenseitig untrennbar miteinander verschweißt sind.

2. Verschluß nach Anspruch 1, dadurch gekennzeichnet, daß an der Stirnseite (4) der Kappe (3) mehrere Durchstichöffnungen vorgesehen sind.

3. Verschluß nach Anspruch 1 oder 2, dadurch gekennzeichnet, daß mindestens eine Durchstichöffnung (6) an der Stirnseite (4) der Kappe (3) mit der elastischen Dichtschicht (5) derart verschlossen ist, daß der in diese Öffnung (6) hineinragende Dichtschichtabschnitt (5') mit der äußeren Stirnfläche (24) der Kappenstirnseite (4) eine praktisch ebene Fläche bildet.

4. Verschluß nach einem der Ansprüche 1 bis 3, dadurch gekennzeichnet, daß der oder die in die Durchstichöffnung(en) (6) der Kappe (3) hineinragende(n) Abschnitt(e) (5') der elastischen Dichtschicht am Öffnungs-Wandbereich (9) fest anliegt(en) und dort untrennbar mit der Kappe (3) verschweißt ist (sind).

5. Verschluß nach einem der Ansprüche 1 bis 4, dadurch gekennzeichnet, daß die Stirnseite (4) der Kappe (3) etwa im Bereich der Flaschenmündung (10) napfartig und von der Flaschenmündung wegweisend ausgebildet ist und daß bei der Kappe (3) an ihrer der Flasche (1) zugewandten Seite ein Befestigungsflansch (13) vorgesehen ist, der mit einem Gegenflansch (14) der Medikamentenflasche (1) durch Verschweißen dichtend verbindbar ist.

6. Verschluß für eine Medikamentenflasche nach einem der Ansprüche 1 bis 5, deren Mündung im unbenutzten Zustand mittels eines Flaschendeckels verschlossen ist, der mit der Flaschenmündung in Verbindung steht, dadurch gekennzeichnet, daß die dem Flaschendeckel (21) zugewandte Seite der elastischen Dichtschicht (5) so an diesen Flaschendeckel (21) angepaßt ist, daß sie im unbenutzten Zustand dichtend an dem Flaschendeckel (21) anliegt.

7. Verschluß nach einem der Ansprüche 1 bis 6, dadurch gekennzeichnet, daß die Durchstichöffnung(en) und der oder die sie ausfüllende(n) Abschnitt(e) (5') der elastischen Dichtschicht (5) mit einer abziehbaren, im ungebrauchten Zustand den Umgebungs-Bereich (19) der Durchstichöffnung(en) (6) dicht verschließenden Abdeckung (25) versehen ist (sind).

8. Verschluß nach Anspruch 7, dadurch gekennzeichnet, daß die Abdeckung(en) (25) jeweils rund um eine oder mehrere Durchstichöffnung(en) (6) aufgeklebt ist (sind).

9. Verschluß nach Anspruch 7, dadurch gekennzeichnet, daß die Abdeckung(en) rund um eine oder mehrere Durchstichöffnung(en) (6) auf die Kappe (3) aufgeschweißt ist (sind).

10. Verschluß nach einem der Ansprüche 7 bis 9, dadurch gekennzeichnet, daß die Abdeckung (25) wenigstens einer Durchstichöffnung (6) aus PE (Polyethylen) oder aus PP (Polypropylen) besteht.

11. Verschluß nach einem der Ansprüche 7 bis 9, dadurch gekennzeichnet, daß die Abdeckung wenigstens einer Durchstichöffnung (6) aus zumindest einem Aluminiumblättchen oder einer Aluminiumfolie besteht(en).

12. Verschluß nach einem der Ansprüche 1 bis 11, dadurch gekennzeichnet, daß der Werkstoff der Dichtschicht (5) farblich gegenüber dem Werkstoff der Kappe (3) kontrastiert.

13. Verfahren zum Herstellen eines Verschlusses (2) für Medikamentenflaschen (1), bei dem in einem ersten Herstellungsschritt die Kappe (3) des Verschlusses hergestellt und anschließend in einem weiteren Herstellungschritt in demselben Werkzeug oder einem Teil davon eine elastische Dichtschicht in die Kappe eingespritzt wird, zum Herstellen des Verschlusses nach einem der Ansprüche 1 bis 12, wobei beim ersten Herstellungsschritt die Kappe (3) mit wenigstens einer Durchstichöffnung (6) hergestellt und in einem zweiten Herstellungsschritt die elastische Dichtschicht (5) in die Kappe (3) sowie deren Durchstichöffnung (6) eingebracht wird, dadurch gekennzeichnet, daß die Dichtschicht (5) und die Kappe (3) kappeninnenseitig untrennbar miteinander verschweißt werden.

## Claims

1. A closure (2) for a medical bottle (1) including a cup-shaped cap (3) connectable to the bottle, said cap having at the end face thereof at least one perforable opening (6), further including a perforable sealing element (5) which is situated in the cap (3) and consists of an elastic sealing layer (5) projecting into the perforable opening (6), as well as at least one removable covering (25) overlapping the perforable opening (6) at the outer end face (24) of the closure (2), the cap consisting of a material of less elasticity than the sealing element. characterized in that on the inside of the cap the cap (3) and the sealing layer (5) are inseparably welded together.

2. A closure as claimed in claim 1, characterized in that a plurality of perforable openings are provided at the end face (4) of the cap (3).

3. A closure as claimed in claim 1 or claim 2, characterized in that at least one perforable opening (6) at the end face (4) of the cap (3) is closed by the elastic sealing layer (5) in such a way that the sealing layer portion (5') projecting into said opening (6) forms a practically plane surface with the outer end surface (24) of the end face (4) of the cap.

4. A closure as claimed in any one of claims 1 to 3, characterized in that the portion(s) (5') of the elastic sealing layer projecting into the perforable opening(s) (6) of the cap (3) sit(s) close against the wall area (9) of said opening(s) and is (are) inseparably welded to the cap (3) there.

5. A closure as claimed in any one of claims 1 to 4, characterized in that near the bottle mouth (10) or thereabouts the end face (4) of the cap (3) is configured to be cup-like and pointing away from the bottle mouth, and that the cap (3) side facing the bottle (1) is provided with an attachment flange (13) sealingly joinable to a companion flange (14) of the medical bottle (1) by welding.

6. A closure for a medical bottle as claimed in any one of claims 1 to 5, in the unused condition the mouth thereof being closed by means of a bottle top connected to the mouth of the bottle, characterized in that the side of the elastic sealing layer (5) facing the bottle top (21) is adapted to said bottle top (21) in such a way as to sit sealingly thereagainst in the unused condition.

7. A closure as claimed in any one of claims 1 to 6, characterized in that the perforable opening(s) and the portion(s) (5') of the elastic sealing layer (5) filling the same is (are) provided with a peelable covering (25) sealing the surrounding area (19) of the perforable opening(s) (6) in the unused condition.

8. A closure as claimed in claim 7, characterized in that the covering(s) (25) is (are) adhesively secured around one or several perforable opening(s) (6).

9. A closure as claimed in claim 7, characterized in that the covering(s) is (are) welded onto the cap (3) around one or several perforable opening(s).

10. A closure as claimed in any one of claims 7 to 9, characterized in that the covering (25) of at least one perforable opening (6) consists of PE (polyethylene) or of PP (polypropylene).

11. A closure as claimed in any one of claims 7 to 9, characterized in that the covering of at least one perforable opening (6) consist(s) of at least one aluminium lamina or aluminium foil.

12. A closure as claimed in any one of claims 1 to 11, characterized in that the material of the sealing layer (5) forms a colour contrast to the material of the cap (3).

13. A method of manufacturing a closure (2) for medical bottles (1), wherein in a first production step the cap (3) of the closure is produced and then in a further production step in the same mould or a part thereof an elastic sealing layer is injected into the cap, for producing the closure as claimed in any one of claims 1 to 12, in the first production step the cap (3) being produced with at least one perforable opening (6) and in a second production step the elastic sealing layer (5) being introduced into the cap (3) and perforable opening (6) thereof, characterized in that on the inside of the cap the sealing layer (5) and the cap (3) are inseparably welded together.

## Revendications

1. Fermeture (2) pour un flacon (1) à usage médical, comprenant un bouchon (3) en forme de pot, qui peut être assemblé au flacon et présente au moins une ouverture de perforation (6) sur son côté frontal, un élément d'étanchéité (5) perforable, qui se trouve dans le bouchon (3) et est constitué d'une couche d'étanchéité élastique (5) pénétrant dans l'ouverture de perforation (6), ainsi qu'au moins un élément de recouvrement (25), qui peut être retiré et recouvre l'ouverture de perforation (6) sur la face frontale extérieure (24) de la fermeture (2), le bouchon étant réalisé en un matériau moins élastique que l'élément d'étanchéité, **caractérisée** en ce que le bouchon (3) et la couche d'étanchéité (5) sont soudés l'un à l'autre de manière inséparable sur le côté intérieur du bouchon.

2. Fermeture selon la revendication 1, **caractérisée** en ce que plusieurs ouvertures de perforation sont prévues sur le côté frontal (4) du bouchon (3).

3. Fermeture selon la revendication 1 ou 2, **caractérisée** en ce qu'au moins une ouverture de perforation (6) sur le côté frontal (4) du bouchon (3) est fermée par la couche d'étanchéité élastique (5) de telle sorte que la partie de couche d'étanchéité (5') qui pénètre dans cette ouverture (6) forme une surface pratiquement plane avec la face frontale extérieure (24) du côté frontal (4) du bouchon.

4. Fermeture selon l'une quelconque des revendications 1 à 3, **caractérisée** en ce que la ou les parties (5') de la couche d'étanchéité élastique qui pénètre(nt) dans la ou les ouvertures de perforation (6) du bouchon (3) s'applique(nt) fixement contre la région de paroi d'ouverture (9) et y est ou sont soudées de manière inséparable au bouchon (3).

5. Fermeture selon l'une quelconque des revendications 1 à 4, **caractérisée** en ce que le côté frontal (4) du bouchon (3) est configuré, dans la région du goulot (10) du flacon, approximativement en forme de coupelle et en éloignement du goulot du flacon, et en ce qu'est prévue, sur le côté du bouchon (3) qui est tourné vers le flacon (1), une bride de fixation (13) qui peut être hermétiquement assemblée, par soudage, à une bride complémentaire (14) du flacon (1) à usage médical.

6. Fermeture selon l'une quelconque des revendications 1 à 5, pour un flacon à usage médical dont le goulot est, à l'état non utilisé, fermé par un couvercle de flacon qui est assemblé au goulot du flacon, **caractérisée** en ce que le côté de la couche d'étanchéité élastique (5) qui est tourné vers le couvercle de flacon (21) est adapté à ce couvercle de flacon (21) de telle sorte qu'à l'état non utilisé, il s'applique hermétiquement contre le couvercle de flacon (21).

7. Fermeture selon l'une quelconque des revendications 1 à 6, **caractérisée** en ce que la ou les ouvertures de perforation, et la ou les parties (5') de la couche d'étanchéité élastique (5) qui remplissent cette ou ces ouvertures, est ou sont pourvues d'un élément de recouvrement (25) qui peut être retiré et qui, à l'état non utilisé, ferme hermétiquement la région environnante (19) de l'ouverture ou des ouvertures de perforation (6).

8. Fermeture selon la revendication 7, **caractérisée** en ce que le ou chaque élément de recouvrement (25) est collé circulairement autour d'une ou plusieurs ouvertures de perforation (6).

9. Fermeture selon la revendication 7, **caractérisée** en ce que le ou chaque élément de recouvrement est soudé circulairement autour d'une ou plusieurs ouvertures de perforation (6) sur le bouchon (3).

10. Fermeture selon l'une quelconque des revendications 7 à 9, **caractérisée** en ce que l'élément de recouvrement (25) d'au moins une ouverture de perforation (6) est réalisé en polyéthylène ou en polypropylène.

11. Fermeture selon l'une quelconque des revendications 7 à 9, **caractérisée** en ce que l'élément de recouvrement d'au moins une ouverture de perforation (6) est constitué d'au moins une lamelle ou feuille d'aluminium.

12. Fermeture selon l'une quelconque des revendications 1 à 11, **caractérisée** en ce que la couleur du matériau de la couche d'étanchéité (5) contraste avec celle du matériau du bouchon (3).

13. Procédé de fabrication d'une fermeture (2) pour des flacons (1) à usage médical, selon lequel, au cours d'une première étape de fabrication, on réalise le bouchon (3) de la fermeture puis, au cours d'une autre étape de fabrication, dans le même moule ou dans une partie de ce moule, on injecte une couche d'étanchéité élastique dans le bouchon, pour la fabrication de la fermeture selon l'une quelconque des revendications 1 à 12, le bouchon (3) étant, lors de la première étape de fabrication, réalisé avec au moins une ouverture de perforation (6), tandis qu'au cours d'une seconde étape de fabrication, la couche d'étanchéité élastique (5) est introduite dans le bouchon (3) ainsi que dans son ouverture de perforation (6), **caractérisé** en ce que la couche d'étanchéité (5) et le bouchon (3) sont soudés l'un à l'autre de manière inséparable sur le côté intérieur du bouchon.
